# EUROPÄISCHE PATENTSCHRIFT

(11) **EP 0 729 982 B1**
(45) Veröffentlichungstag und Bekanntmachung des Hinweises auf die Patenterteilung: **18.08.1999**
(21) Anmeldenummer: 96101980.9
(22) Anmeldetag: 12.02.1996
(51) Int. Cl.: C08F 110/06, C08F 2/00, C08F 2/06, C07C 7/04, B01D 3/14, C08J 11/02

(54) **Verfahren zur Reinigung von nicht umgesetztem Propylen bei der Herstellung von Polypropylen**
Process for the purification of non-reacted propylene in the production of polypropylene
Procédé de purification de propylène n'ayant pas reagi dans la préparation de polypropylène

(30) Priorität: 28.02.1995 DE 19506901
(43) Veröffentlichungstag der Anmeldung: 04.09.1996
(73) Patentinhaber: TARGOR GmbH, 55116 Mainz (DE)
(72) Erfinder: Voigt, Hartmut, Dipl.-Ing., D-61462 Königstein (DE)

(56) Entgegenhaltungen:
- EP-A- 0 586 244
- WO-A-93/24533
- DE-B- 1 173 890
- GB-A- 2 160 543
- US-A- 3 652 515
- US-A- 4 692 501

## Beschreibung

Die Erfindung betrifft ein Verfahren zur Reinigung von Propylen bei der Herstellung von Polypropylen.

Polypropylen wird nach dem Stand der Technik hauptsächlich in Suspension hergestellt. Dabei werden die Produkte, die erhaltenen Polymere, entweder in einem flüssigen Einsatzstoff, der bei neueren Verfahren dem Monomeren entspricht, suspendiert oder in dem gasförmigen Einsatzstoff in einem Reaktor in Suspension gehalten. Die Reaktionswärme wird wahlweise durch gekühlte Oberflächen am Reaktor, über einen umlaufenden Teilstrom oder durch Verdampfen und Kondensation des Reaktorinhaltes abgeführt.
Die Polymerisation erfolgt mit Hilfe von Katalysatoren, deren Weiterentwicklung im Laufe der Zeit zu ständig höheren Polymerausbeuten führte.
Typische Polypropylen-Produkte haben einen weitgehend isotaktischen Molekülaufbau mit geringen Anteilen an ataktischem Polymer und niedermolekularen Ölen.

Die Produkte werden auch durch den Einbau von Comonomeren, insbesondere durch Ethylen modifiziert, wobei die Comonomere statistisch verteilt eingebaut werden oder auch in Sequenzen vorliegen können. Die Entwicklung der Katalysatoren im Hinblick auf besonders hohe Ausbeuten brachte auf der einen Seite den Vorteil, Katalysatorreste im Fertigprodukt belassen zu können, auf der anderen Seite sind jedoch diese modernen Katalysatoren anfälliger für Nebenreaktionen, die mit Störkomponenten ablaufen, welche mit Roh- oder Hilfsstoffen oder über Undichtigkeiten der Anlage in das Produktionssystem eindringen. Solche Störkomponenten sind z.B. Sauerstoff, Stickstoff, Wasserstoff, Wasser, Kohlendioxid, Kohlenmonoxid, reaktive Kohlenwasserstoffe wie Propadien, Allen, Methylacetylen usw. Aber auch inerte Kohlenwasserstoffe wie Methan, Ethan, Propan usw. können die Polymerisation stören.
Die Reinheit der eingesetzten Roh- bzw. Hilfsstoffe hat also bei der Nutzung moderner Katalysatorsysteme eine sehr große Bedeutung. Dabei werden heute Rohstoffe eingesetzt, die einerseits durch spezielle Maßnahmen einen hinreichend niedrigen Gehalt an Störkomponenten aufweisen, auf der anderen Seite jedoch wird häufig ein Propylen als Rohstoff (Frischpropylen) für die Polymerisation eingesetzt, welches noch gewisse Gehalte an Propan aufgrund seines Herstellprozesses aufweist.

Typischerweise wird Frischpropylen, mit einem Gehalt von 5 bis 10 % (Gew.) Propan eingesetzt, wobei dieses chemisch inerte Propan im Reaktionsraum auf 20 bis 30 % angereichert wird. Diese Propananreicherung hat lediglich zur Folge, daß das im Reaktionsraum des Reaktors wirksame Propylen Monomer mit einer niedrigeren Konzentration am Katalysator wirksam wird und die Polymerisationsgeschwindigkeit entsprechend verlangsamt wird. Höhere Propylengehalte führen also bei sonst gleichen Bedingungen zu einer höheren Produktionskapazität vorhandener Reaktionssysteme.
Das im Reaktionsraum angereicherte Propan wird üblicherweise zusammen mit dem nicht umgesetztem Propylen vom Produkt abgetrennt und in einer nachgeschalteten Destillationskolonne, die im folgenden als C₃-Trennkolonne bezeichnet wird, aufgetrennt. Dabei fällt im Sumpf der Kolonne das Propan zur weiteren Entsorgung an, während am Kopf ein gereinigtes Propylen gewonnen wird, das in den Reaktionsraum zurückgeführt wird und das einen Propangehalt aufweist, der niedriger ist als derjenige, der im Reaktionsraum vorliegt. Er kann jedoch durchaus höher sein als derjenige, der mit dem Frischpropylen in das System eingesetzt wird. Die nach dem Stand der Technik verwendeten C₃-Trennkolonnen werden so ausgelegt und betrieben, daß die Propylenverluste zusammen mit dem ausgeschleusten Propan, die Propananreicherung im Reaktionssystem und die aufgewendeten Kosten insgesamt in einem optimalen Zusammenhang stehen.

Nachteil des beschriebenen Verfahrens ist, daß das von außen zugeführte Frischpropylen in der Qualität wie angeliefert direkt in den Reaktionsraum gelangt. Wie bereist oben ausgeführt, reduziert das im Propylen enthaltene Propan einerseits die Propylenkonzentration am Katalysator, andererseits bringt das Frischpropylen auch höhermolekulare Kohlenwasserstoffe, sogenannte Green Oils, in den Reaktionsraum ein, die z.T. als Polymerisationsgifte wirken. Deshalb ist man bemüht, Roh- bzw Hilfsstoffe einzusetzen, die einen hinreichend niedrigen Gehalt an Störkomponenten aufweisen, die dadurch aber auch teurer sind als nicht behandelte Roh- bzw Hilfsstoffe.
Eine Möglichkeit, die Verwendung eines gereinigten Rohstoffes zu umgehen, wird in der EP OS 0006619, "Verfahren zur Reinigung von Propylen für ein Massepolymerisationsverfahren", beschrieben. Nach diesem Verfahren wird ein ungereinigter Rohstoff eingesetzt und bei der Herstellung des Polymers gereinigt. Dabei wird das erhaltene kristalline Polypropylen durch Extraktion der vom Reaktor kommenden Polymersuspension mit einem flüssigen Kohlenwasserstoff von löslichen Polymeren und Katalysatorbestandteilen abgetrennt, und zwar dadurch, daß die Polymersuspension vor der Extraktion soweit abgekühlt wird, daß keine nennenswerte Polymerisation mehr stattfindet und daß das zu reinigende Frischpropylen der Extraktionsflüssigkeit zugegeben wird. Die Trennung des Feststoffs von der Flüssigphase erfolgt dabei durch Sedimentation in der Extraktionskolonne. Die Extraktionsflüssigkeit wird anschließend zusammen mit der Suspensionsflüssigkeit einer mehrstufigen Destillation unterworfen, in der Leichtsieder, Propylen und Schwersieder voneinander getrennt werden. Das gereinigte Propylen wird anschließend dem Reaktor zugeführt. Es enthält das Propylen aus dem Frischpropylen und aus dem nicht umgesetztem Propylen aus dem Reaktor. Laut EP OS 0006619 enthält das gereinigte Propylen 8 ppm Propadien, 3 ppm Methylacetylen, 3 ppm Sauerstoff, <3 ppm Wasser, <1 ppm Acetylen, <1 ppm Kohlenmonoxid und <1 ppm Kohlendioxid. Green Oils werden nicht erwähnt.
Nachteilig an diesem Verfahren ist, daß gegenüber dem Stand der Technik keine verbesserte Propanabtrennung und keine Abtrennung von Green Oils erreicht wird. Die eigentliche Zielsetzung der EP OS 0006619 ist die Entferung von (Co)-Katalysatorbestandteilen aus dem hergestellten Polypropylen. Durch Reaktion der zu entfernenden (Co)-Katalysatorbestandteile mit dem Frischpropylen wird das Frischpropylen zwar auch gereinigt, eine verbesserte Propan- und Green Oils-Abtrennung kann jedoch nicht erreicht werden, da die nachgeschaltete C₃-Trennkolonne in üblicher Art und Weise betrieben wird. Die Reinigung kann hier nur polare Verunreinigungen wie Wasser, CO₂ etc. entfernen. Nachteilig ist außerdem, daß eine Extraktionsflüssigkeit - die auch aus Propylen bestehen kann - im Kreis gefahren werden muß und daß das Absetzen des kristallinen Polypropylens behindert wird, da die Extraktionsflüssigkeit in der Extraktionskolonne entgegen der Schwerkraft von unten nach oben geführt werden muß.

Zusammenfassend wird festgestellt, daß die Polymerisationsverfahren nach dem Stand der Technik nicht in der Lage sind, propanhaltiges und Green Oils enthaltendes Propylen soweit zu reinigen, daß es für die Polymerisation mit einem modernen Katalysatorsystem geeignet ist.

Der Erfindung lag somit die Aufgabe zugrunde, ein Verfahren zu entwickeln, das eine verbesserte Ausschleusung von Propan, Störkomponenten und Green Oils aus dem Reaktionssystem erlaubt.

Erfindungsgemäß geschieht dies dadurch, daß das nicht umgesetzte Propylen unter Verwendung von Frischpropylen als Rücklaufflüssigkeit in einer dem Reaktor vorgeschalteten Destillation behandelt wird und der Destillation gereinigtes Propylen als Destillationsprodukt entnommen wird.

Weitere Ausführungsformen der Erfindung ergeben sich aus den Ansprüchen 2 bis 7. Bei diesem als Reverse Feed Process (RFP) bezeichneten Verfahren wird das Frischpropylen im Gegenstrom zum Weg des nicht umgesetzten Propylens geführt und in einer dem Reaktor vorgeschalteten Destillation in an sich bekannter Weise der Reinigung unterworfen. Darüber hinaus unterstützt das Frischpropylen als Destillationshilfsmittel die Abtrennung des Propans aus dem nicht umgesetzten Propylen.
Die Erfindung bezieht sich auf alle Arten von Verfahren zur Herstellung von Polypropylen aus Frischpropylen, die mit einer C₃-Trennkolonne ausgerüstet sind. Es findet in erster Linie bei der Polymerisation im flüssigen Monomeren Anwendung, eignet sich aber ebenso bei der Polymerisation in der Gasphase. Bei dem erfindungsgemäßen Verfahren wird das Frischpropylen nicht direkt in den Reaktionsraum eingeführt, sondern vielmehr als Rücklaufflüssigkeit in die C₃-Trennkolonne eingesetzt. Dadurch wird im allgemeinen die Trennleistung der Kolonne verbessert, d.h. sie kann gegenüber dem Normalbetrieb mit geringerem Energieeinsatz die gleiche Trennleistung erbringen oder sie kann bei gleichem Energieeinsatz eine verbesserte Trennleistung erbringen, d.h. die Propylenverluste im Sumpf verringern und/oder den Propangehalt im Reaktionssystem absenken. Zusätzlich zu der oben beschriebenen Verbesserung wird gleichzeitig das Frischpropylen der Trennwirkung der Kolonne unterworfen und gibt entsprechend schwersiedende Produkte an das Sumpfprodukt ab, d.h. sie gelangen nicht in das Reaktionssystem.

Bei einer möglichen Ausführungsform des erfindungsgemäßen Verfahrens wird das eingesetzte Frischpropylen zusammen mit dem Kolonnenrücklauf auf den Kopf der Kolonne aufgegeben und das nicht umgesetzte Propylen der Kolonne in der oberen Hälfte zugeführt. In diesem Fall wird die Propanabtrennung verbessert und es werden schwersiedende Komponenten des Frischpropylens insbesondere solche mit 4 und mehr C-Atomen, mit Abreicherungsgraden von mehr als 90 % von dem Reaktionssystem zurückgehalten.

Bei einer weiteren Ausführung des erfindungsgemäßen Verfahrens wird das Frischpropylen an einer Stelle unterhalb des Kopfes der Kolonne eingeführt. Bei nicht veränderter Auslegung der Trennkolonne wird hierdurch die Propanabtrennung gegenüber dem Normalbetrieb einerseits geringfügig verschlechtert. Dagegen ist diese Variante aber auch in der Lage, im Frischpropylen vorhandene Störkomponenten mit 3 C-Atomen wie Propadien und Methylacetylen mit guten Abreicherungsgraden vom Reaktionssystem fernzuhalten.

Mit einer weiteren Ausführungsform kann das erfindungsgemäße Verfahren auch dazu genutzt werden, im Frischpropylen etwa vorhandene leichtsiedende Störkomponenten von dem Reaktionssystem fernzuhalten. Hierzu wird das Destillationsprodukt der Destillationskolonne als Seitenstrom entnommen, wodurch in dem darüberliegenden Kolonnenkopfteil eine Anreicherung von sog. inerten, d.h. leichtsiedenden Störkomponenten erfolgt, die über den Kolonnenkopf aus dem Reaktionssystem ausgeschleust werden können. Diese Ausführung ist besonderes dazu geeignet, relativ unreine und preiswerte Propylenqualitäten des Typs Chemical Grade direkt in Polypropylenanlagen einzusetzen, die mit einer entsprechend ausgerüsteten und erfindungsgemäß in die Verfahrensführung eingebundenen C₃-Kolonne betrieben werden.

Die Vorteile des erfindungsgemäßen Verfahrens bestehen darin, daß preisgünstiges Propylen eingesetzt werden kann, daß eine Extraktionsstufe vermieden wird und daß Green Oils, Leicht-, Schwersieder und Propan nahezu quantitativ abgetrennt werden können. Dadurch werden die Rohstoff- und Investitionskosten gesenkt, die Ausbeute der Polymerisation erhöht und insgesamt die Wirtschaftlichkeit der Polypropylenherstellung verbessert.

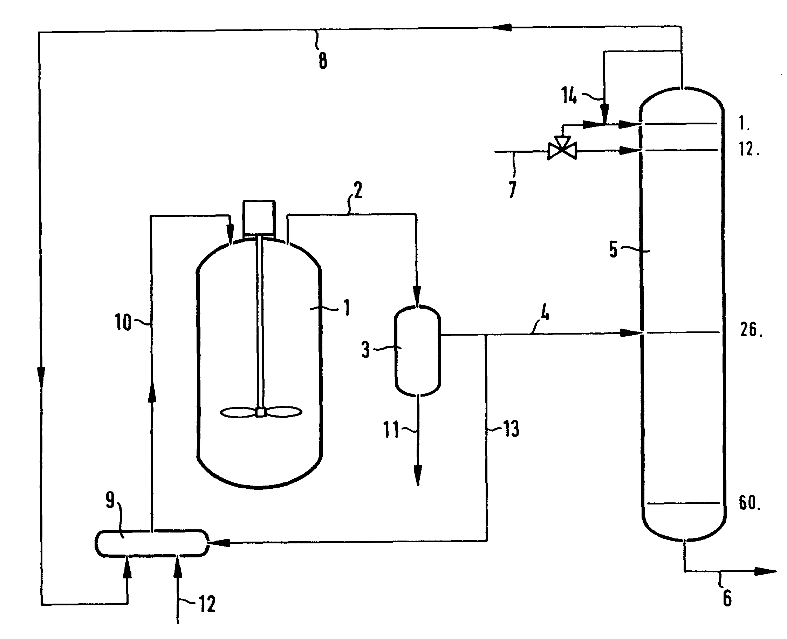

Im folgenden wird das erfindungsgemäße Verfahren anhand des in der einzigen Figur dargestellten Fließbildes näher erläutert.

In einem Reaktor 1 wird Propylen in Anwesenheit von Katalysatoren in flüssigem Propylen polymerisiert. Dem Reaktor 1 wird eine Suspension von Polypropylen in flüssigem Propylen entnommen und über eine Leitung 2 einem Separator 3 zugeführt. In dem Separator 3 wird das feste Polypropylen von der flüssigen Phase getrennt und über eine Leitung 11 abgezogen.
Ein Teil, vorzugsweise 20 % (Gew.), der verbliebenen flüssigen Phase wird über eine Leitung 4 auf den 26. theoretischen Boden einer Destillationskolonne 5 - beispielsweise einer Füllkörperkolonne mit einem Durchmesser von 1850 mm, einer Höhe von 33500 mm und 60 theoretischen Böden (von oben nach unten steigend durchnumeriert) - aufgegeben. Der Rest wird über eine Leitung 13 und über ein Vorratsgefäß 9 in den Reaktor 1 zurückgeführt. Entsprechend der Erfindung wird Frischpropylen auf den 12. theoretischen Boden der Destillationskolonne 5 über eine Leitung 7 aufgegeben. Es kann aber auch zusammen mit der Rücklaufflüssigkeit dem Kolonnenkopf am 1. theoretischen Boden zugeführt werden. Aus dem Sumpf der Destillationskolonne 5 wird über eine Leitung 6 eine Mischung aus Propylen, Propan und Schwersiedern wie Green Oils und höhermolekularen Kohlenwasserstoffen abgezogen. Die Destillationskolonne 5 wird bei einer Temperatur von 35 °C und einem Druck von 20 bar betrieben, wobei die Rücklaufflüssigkeit der Destillationskolonne 5 über eine Leitung 14 auf den Kolonnenkopf zurückgeführt wird. Über den Kolonnenkopf geht eine Mischung bestehend im wesentlichen aus Propylen und Propan mit geringen Anteilen an Leichtsiedern durch eine Leitung 8 dem Vorratsgefäß 9 zu, um von dort durch eine Leitung 10 dem Reaktor 1 zugeführt zu werden. Das Vorratsgefäß 9 dient als Zwischen- oder Puffergefäß und weist einen zusätzlichen Zulauf 12 auf.
Bei einer Durchführung des Verfahrens in an sich bekannter Weise ergab eine gaschromatographische Analyse von Frischpropylen, Sumpfprodukt aus der Destillationskolonne 5 und Kopfprodukt der Destillationskolonne 5 folgende

### Zusammensetzung:

Frischpropylen: 95,02 % Propylen, 4,68 % Propan, 200 ppm Ethan, 45 ppm Butane, 600 ppm Butene, 180 ppm gesättigte und ungesättigte C₅-Kohlenwasserstoffe, 980 ppm gesättigte und ungesättigte C₆-Kohlenwasserstoffe.
Sumpfprodukt: 80,7 % Propan, 17,7 % Propylen, 3,6 % höhermolekulare gesättigte und ungesättigte Kohlenwasserstoffe.
Kopfprodukt: 86,2 % Propylen, 13,5 % Propan, 4 ppm Butene, Butane sowie Green Oils waren nicht nachweisbar.
Das dem Reaktor 1 zugeführte Propylen enthält also keine höhermolekularen Kohlenwasserstoffe. Das erfindungsgemäße Verfahren erlaubt also, die im Frischpropylen enthaltenen Green Oils quantitativ abzutrennen.

### Vergleichsbeispiel:

In einer Polymerisationsanlage, die entsprechend dem Stand der Technik betrieben wird, wird Propylen zu Polypropylen polymerisiert. Dem Verfahren liegt ebenfalls das in der Figur dargestellte Fließbild zugrunde, jedoch wird das Frischpropylen über den Zulauf 12 des Puffergefäßes 9 dem System zugeführt und nicht über die Leitung 7 der Destillationskolonne 5.

Es wurden Proben wie im Beispiel vorher gezogen und gaschromatographisch analysiert:

Sumpfprodukt: 80.7 % Propan, 17.7 % Propen und 1.6 % höhermolekulare ungesättigte Kohlenwasserstoffe.

Frischpropylen: 95.02 % Propen, 4.68 % Propan, 200 ppm Ethan, 45 ppm Butane, 600 ppm Butene, 180 ppm gesättigte und ungesättigte C5 Kohlenwasserstoffe und 980 ppm gesättigte und ungesättigte C6 Kohlenwasserstoffe.

Kopfprodukt: 86.8 % Propylen, 12.5 % Propan, 1270 ppm Ethan, 106 ppm Butane, 1420 ppm Butene, 430 ppm gesättigte und ungesättigte C5 Kohlenwasserstoffe, 2320 ppm gesättigte und ungesättigte C6 Kohlenwasserstoffe.

Das dem Reaktor 1 zugeführte Propylen enthält also im Gegensatz zum erfindungsgemäßen Verfahren höhermolekulare Kohlenwasserstoffe.

## Patentansprüche

1. Verfahren zur Reinigung von nicht umgesetztem Propylen aus der katalytischen Herstellung von Polypropylen aus Frischpropylen, wobei das nicht umgesetzte Propylen von der aus dem Reaktor kommenden Polymersuspension abgetrennt wird, dadurch gekennzeichnet, daß das nicht umgesetzte Propylen unter Verwendung von Frischpropylen als Rücklaufflüssigkeit in einer dem Reaktor vorgeschalteten Destillation behandelt wird und der Destillation gereinigtes Propylen als Destillationsprodukt entnommen wird.

2. Verfahren nach Anspruch 1, dadurch gekennzeichnet, daß das nicht umgesetzte Propylen zur Destillation einer Destillationskolonne in der oberen Hälfte zugeführt wird und daß das Frischpropylen der Destillationskolonne oberhalb der Zuführung des nicht umgesetzten Propylens zugegeben wird.

3. Verfahren nach Anspruch 1, dadurch gekennzeichnet, daß das Frischpropylen einer Destillationskolonne zusammen mit deren Rücklaufflüssigkeit am Kopf zugegeben wird und daß das nicht umgesetzte Propylen der Destillationskolonne in deren oberer Hälfte zugegeben wird.

4. Verfahren nach Anspruch 1, dadurch gekennzeichnet, daß das Frischpropylen einer Destillationskolonne unterhalb des Kolonnenkopfes und das nicht umgesetzte Propylen in der oberen Hälfte der Destillationskolonne, jedoch unterhalb des Aufgabepunktes des Frischpropylens zugeführt wird.

5. Verfahren nach Anspruch 1, dadurch gekennzeichnet, daß das Frischpropylen einer Destillationskolonne am Kopf zugeführt wird.

6. Verfahren nach Anspruch 2, dadurch gekennzeichnet, daß das nicht umgesetzte Propylen unterhalb des zweiten Bodens der Destillationskolonne zugeführt wird und daß das Frischpropylen der Destillationskolonne am zweiten Boden zugeführt wird.

7. Verfahren nach Anspruch 4, dadurch gekennzeichnet, daß der Destillationskolonne oberhalb der Zuführung des nicht umgesetztem Propylens und unterhalb der Zuführung des Frischpropylens das Destillationsprodukt als Seitenstrom entnommen wird.

## Claims

1. A process for purifying unreacted propylene from the catalytic preparation of polypropylene from fresh propylene, with the unreacted propylene being separated from the polymer suspension coming from the reactor, which comprises treating the unreacted propylene in a distillation upstream of the reactor using fresh propylene as runback liquid and taking purified propylene from the distillation as distillation product.

2. The process as claimed in claim 1, wherein the unreacted propylene is, for the purposes of distillation, fed to a distillation column in the upper half and wherein the fresh propylene is supplied to the distillation column above the feed point for the unreacted propylene.

3. The process as claimed in claim 1, wherein the fresh propylene is supplied to a distillation column at the top together with the runback liquid of the column and wherein the unreacted propylene is supplied to the distillation column in its upper half.

4. The process as claimed in claim 1, wherein the fresh propylene is fed to a distillation column below the top of the column and the unreacted propylene is fed to the upper half of the distillation column, but below the point at which the fresh propylene is supplied.

5. The process as claimed in claim 1, wherein the fresh propylene is fed to a distillation column at the top.

6. The process as claimed in claim 2, wherein the unreacted propylene is fed in below the second plate of the distillation column and wherein the fresh propylene is fed to the distillation column at the second plate.

7. The process as claimed in claim 4, wherein the distillation product is taken as a branch stream from the distillation column above the feed point for the unreacted propylene and below the feed point for the fresh propylene.

## Revendications

1. Procédé de purification de propylène n'ayant pas réagi issu de la préparation catalytique de polypropylène à partir de propylène frais, dans lequel le propylène n'ayant pas réagi est séparé de la suspension de polymère provenant du réacteur, caractérisé en ce que le propylène n'ayant pas réagi est traité dans une distillation montée en amont du réacteur, avec utilisation de propylène frais comme liquide de reflux, et en ce que le propylène purifié par la distillation est prélevé sous la forme d'un produit de distillation.

2. Procédé suivant la revendication 1, caractérisé en ce que le propylène n'ayant pas réagi est amené à la distillation d'une colonne de distillation, dans la moitié supérieure, et en ce que le propylène frais est amené à la colonne de distillation au-dessus de l'amenée du propylène n'ayant pas réagi.

3. Procédé suivant la revendication 1, caractérisé en ce que le propylène frais est amené à la tête d'une colonne de distillation conjointement au liquide de reflux de celle-ci et en ce que le propylène n'ayant pas réagi est amené à la colonne de distillation dans sa moitié supérieure.

4. Procédé suivant la revendication 1, caractérisé en ce que le propylène frais est amené à une colonne de distillation en dessous de la tête de colonne et en ce que le propylène n'ayant pas réagi est amené dans la moitié supérieure de la colonne de distillation, mais en dessous du point de chargement du propylène frais.

5. Procédé suivant la revendication 1, caractérisé en ce que le propylène frais est amené à la tête d'une colonne de distillation.

6. Procédé suivant la revendication 2, caractérisé en ce que le propylène n'ayant pas réagi est amené en dessous du deuxième plateau de la colonne de distillation et en ce que le propylène frais est amené au deuxième plateau de la colonne de distillation.

7. Procédé suivant la revendication 4, caractérisé en ce que le produit de distillation est prélevé comme courant latéral de la colonne de distillation, au-dessus de l'amenée du propylène n'ayant pas réagi et en dessous de l'amenée du propylène frais.
